(19) Europäisches Patentamt
European Patent Office
Office européen des brevets

(11) Veröffentlichungsnummer: **0 257 294 B1**

# (12) EUROPÄISCHE PATENTSCHRIFT

(45) Veröffentlichungstag der Patentschrift: **12.09.90**

(51) Int. Cl.[5]: **C07C 255/64**, A01N 37/50

(21) Anmeldenummer: **87110459.2**

(22) Anmeldetag: **20.07.87**

## (54) E/Z-Isomerengemische und reine Z-Isomere von N alpha-(2-Cyan-2-alkoximinoacetyl) aminosäurederivaten und -peptiden.

(30) Priorität: **28.07.86 DE 3625497**

(43) Veröffentlichungstag der Anmeldung:
**02.03.88 Patentblatt 88/9**

(45) Bekanntmachung des Hinweises auf die Patenterteilung:
**12.09.90 Patentblatt 90/37**

(84) Benannte Vertragsstaaten:
**AT BE CH DE ES FR GB GR IT LI NL SE**

(56) Entgegenhaltungen:
**EP-A- 0 201 999**
**EP-A- 0 206 004**
**DE-A- 2 312 956**

(73) Patentinhaber: **BAYER AG,**
**D-5090 Leverkusen 1 Bayerwerk(DE)**

(72) Erfinder: **Lunkenheimer, Winfried, Dr.,**
**Bismarckstrasse 29, D-5600 Wuppertal 1(DE)**
Erfinder: **Berg, Dieter, Dr., Gellertweg 27,**
**D-5600 Wuppertal 1(DE)**
Erfinder: **Brandes, Wilhelm, Dr., Eichendorffstrasse 3,**
**D-5653 Leichlingen(DE)**
Erfinder: **Scheinpflug, Hans, Prof.Dr., Am Thelenhof 15,**
**D-5090 Leverkusen(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue E/Z-Isomerengemische und reine Z-Isomere von $N^{\alpha}$-(2-Cyan-2-alkoximinoacetyl)-aminosäurederivaten und -peptiden, mehrere Verfahren zu ihrer Herstellung und ihre Verwendung als Schädlingsbekämpfungsmittel, insbesondere als Fungizide.

Es ist bereits bekannt, daß bestimmte Verbindungen wie beispielsweise Zink-ethylen-1,2-bis-dithiocarbamat gute fungizide Wirksamkeit besitzen (vgl. z.B. US-PS 2 457 674). Die Wirkung dieser Verbindungen ist jedoch, insbesondere bei niedrigen Aufwandmengen und -konzentrationen, nicht immer voll befriedigend.

Weiterhin sind aus der nachveröffentlichten europäischen Patentanmeldung EP-A 0 201 999 bestimmte fungizid wirksame 2-Cyano-2-Oximino-acetamide bekannt.

Es wurden neue E/Z-Isomerengemische und reine Z-Isomere von $N^{\alpha}$-(2-Cyan-2-alkoximino-acetyl)-aminosäurederivaten und -peptiden der allgemeinen Formel (I)

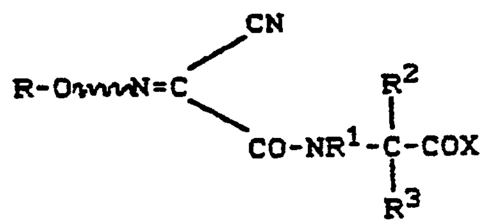

(I)

in welcher

R für geradkettiges oder verzweigtes Alkyl mit 1 bis 8 Kohlenstoffatomen, Alkenyl oder Alkinyl mit jeweils 2 bis 4 Kohlenstoffatomen, für Cyanalkyl mit 1 bis 4 Kohlenstoffatomen, für 1,2,4-Triazol-1-ylalkyl und Pyrazol-1-ylalkyl mit jeweils 1 bis 6 Kohlenstoffatomen im Alkylteil, für gegebenenfalls im Phenylteil einfach bis dreifach, gleich oder verschieden substituiertes Phenylalkyl mit 1 bis 4 Kohlenstoffatomen im Alkylteil steht, wobei als Substituenten vorzugsweise genannt seien: Halogen, Cyano, Nitro, Hydroxy, Alkyl, Alkoxy, Alkylsulfinyl, Alkylsulfonyl und Alkylthio mit jeweils 1 bis 4 Kohlenstoffatomen und gegebenenfalls einfach bis dreifach, gleich oder verschieden durch Halogen substituiertes Phenyl; oder für gegebenenfalls einfach bis dreifach, gleich oder verschieden durch Halogen substituiertes Phenyl; oder für gegebenenfalls einfach bis dreifach, gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen genannt seien;

$R^1$ für Wasserstoff, geradkettiges oder verzweigtes Alkyl mit 1 bis 4 Kohlenstoffatomen oder für jeweils gegebenenfalls einfach bis dreifach, gleich oder verschieden substituiertes Phenyl oder Benzyl steht, wobei als Substituenten vorzugsweise die bei R bereits genannten Phenylsubstituenten in Frage kommen;

$R^2$ für Wasserstoff oder geradkettiges oder verzweigtes Alkyl mit 1 bis 4 Kohlenstoffatomen steht;

$R^3$ für Wasserstoff, geradkettiges oder verzweigtes Alkyl mit 1 bis 6 Kohlenstoffatomen, für geradkettiges oder verzweigtes Hydroxyalkyl mit 1 bis 4 Kohlenstoffatomen, für Alkoxycarbonylalkyl mit jeweils 1 bis 4 Kohlenstoffatomen im Alkoxyteil und im Alkylteil, für Hydroxycarbonylalkyl und Aminocarbonylalkyl mit jeweils 1 bis 4 Kohlenstoffatomen im Alkylteil, für 1,2,4-Triazol-1-ylalkyl, 1,2,4-Triazol-4-ylalkyl, Imidazol-4-ylalkyl und Pyrazol-1-ylalkyl mit jeweils 1 bis 6 Kohlenstoffatomen im Alkylteil, für Cyanalkyl mit 1 bis 4 Kohlenstoffatomen, für Alkenyl oder Alkinyl mit jeweils 2 bis 4 Kohlenstoffatomen, für gegebenenfalls einfach bis dreifach, gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen genannt seien, ferner für gegebenenfalls im Phenylteil einfach bis dreifach, gleich oder verschieden substituiertes Phenyl oder Phenylalkyl mit 1 bis 4 Kohlenstoffatomen im Alkylteil steht, wobei als Substituenten jeweils vorzugsweise die bei R bereits genannten Phenylsubstituenten in Frage kommen, oder für die Gruppierung $R^4\text{–}SO_n\text{–}Z\text{–}$ steht, wobei

$R^4$ für Wasserstoff, geradkettiges oder verzweigtes Alkyl mit 1 bis 4 Kohlenstoffatomen, für gegebenenfalls im Phenylteil einfach bis dreifach, gleich oder verschieden substituiertes Phenylalkyl mit 1 bis 4 Kohlenstoffatomen im Alkylteil steht, wobei als Substituenten die bei R bereits genannten Phenylsubstituenten in Frage kommen;

n für die Zahlen 0, 1 oder 2 steht und

Z für eine geradkettige oder verzweigte Alkylenkette mit 1 bis 4 Kohlenstoffatomen steht;

$R^1$ und $R^3$ gemeinsam mit dem Stickstoffatom und dem Kohlenstoffatom, an die sie gebunden sind, für einen 5- oder 6-gliedrigen Heterocyclus stehen;

$R^2$ und $R^3$ gemeinsam mit dem Kohlenstoffatom, an das sie gebunden sind, für Cycloalkyliden mit 3 bis 6 Kohlenstoffatomen stehen;

X für die Gruppierungen $-OR^{I}$ oder $-NR^{II}R^{III}$ steht, wobei

$R^{I}$ für Wasserstoff, geradkettiges oder verzweigtes Alkyl mit 1 bis 4 Kohlenstoffatomen, Alkenyl oder Alkinyl mit jeweils 2 bis 4 Kohlenstoffatomen steht;

$R^{II}$ für Wasserstoff oder geradkettiges oder verzweigtes Alkyl mit 1 bis 4 Kohlenstoffatomen steht;

$R^{III}$ für Wasserstoff, geradkettiges oder verzweigtes Alkyl mit 1 bis 4 Kohlenstoffatomen, für Alkenyl oder Alkinyl mit jeweils 2 bis 4 Kohlenstoffatomen, für Halogenalkyl mit 1 bis 4 Kohlenstoffatomen und 1 bis 5 gleichen oder verschiedenen Halogenatomen, für Alkoxyalkyl mit jeweils 1 bis 4 Kohlenstoffatomen im Alkoxyteil und im Alkylteil, für Dialkylaminoalkyl mit bis zu 4 Kohlenstoffatomen in den einzelnen Alkylteilen, für Alkoxycarbonylalkyl mit jeweils 1 bis 4 Kohlenstoffatomen im Alkoxyteil und im Alkylteil, Hydroxycarbonylalkyl mit 1 bis 4 Kohlenstoffatomen im Alkylteil, Aminocarbonylalkyl, Alkylaminocarbonylalkyl oder Dialkylaminocarbonylalkyl mit jeweils 1 bis 4 Kohlenstoffatomen in jedem Alkylteil für Cyanalkyl mit 1 bis 4 Kohlenstoffatomen im Alkylteil, gegebenenfalls im Phenylteil einfach bis dreifach, gleich oder verschieden substituiertes Phenylalkyl mit 1 bis 4 Kohlenstoffatomen im Alkylteil oder einfach bis dreifach, gleich oder verschieden substituiertes Phenyl steht, wobei als Substituenten jeweils die bei R bereits genannten Phenylsubstituenten in Frage kommen sowie für gegebenenfalls gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen in Frage kommen;

oder

$R^{II}$ und $R^{III}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann und gegebenenfalls durch Cyano, Halogen, Alkyl mit 1 bis 4 Kohlenstoffatomen, Hydroxycarbonyl, Alkoxycarbonyl mit 1 bis 4 Kohlenstoffatomen im Alkoxyteil, Aminocarbonyl sowie Alkylaminocarbonyl und Dialkylaminocarbonyl mit jeweils 1 bis 4 Kohlenstoffatomen in den einzelnen Alkylteilen substituiert sein kann, ausgenommen die Verbindungen N-(Methoxycarbonylmethyl)-(2-cyano-2-methoximino)-acetamid und N-(Carbamoylmethyl)-(2-cyano-2-methoximino)-acetamid.

Die Verbindungen der Formel (I) besitzen für den Fall, daß $R^2$ und $R^3$ unterschiedliche Bedeutung haben, ein asymmetrisches Kohlenstoffatom; sie können somit auch als optische Isomere (D- und L-Konfiguration) vorliegen, die in unterschiedlichen Mengenverhältnissen anfallen können. Vorwiegend fallen sie als Racemate an.

Weiterhin wurde gefunden, daß man die E/Z-Isomerengemische und reinen Z-Isomeren von $N^{\alpha}$-(2-Cyan-2-alkoximinoacetyl)-aminosäurederivaten und -peptiden der allgemeinen Formel (I)

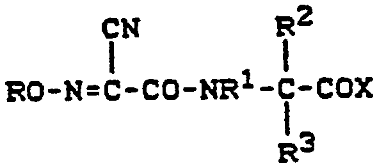

(I)

in welcher

R, $R^1$, $R^2$, $R^3$ und X die oben angegebene Bedeutung haben, ausgenommen die Verbindungen N-(Methoxy-carbonylmethyl)-(2-cyano-2-methoximino)-acetamid und N-(carbamoylmethyl)-(2-cyano-2-methoximino)-acetamid erhält, wenn man die E/Z-Isomerengemische oder die reinen Z-Isomeren der 2-Cyan-2-oximinoessigsäurederivate der allgemeinen Formel (II)

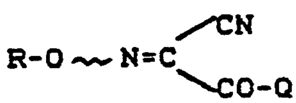

(II)

in welcher

R die oben angegebene Bedeutung hat,

Q für Chlor oder Alkoxy mit 1–4 C-Atomen steht,

mit Aminosäure-Derivaten der allgemeinen Formel (III)

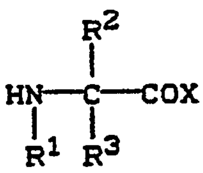

(III)

in welcher
$R^1$, $R^2$, $R^3$ und X die oben angegebenen Bedeutungen haben,
gegebenenfalls in Gegenwart eines Katalysators, gegebenenfalls in Gegenwart eines Säurebindemittels und gegebenenfalls in Gegenwart eines Verdünnungsmittels umsetzt.

Die Verbindungen der allgemeinen Formel (I) fallen beim Einsatz der reinen Z-Isomeren der Formel (II) je nach Reaktionsbedingungen als E/Z-Isomerengemische oder als reine Z-Isomere an.

Die so zu erhaltenden Verbindungen der Formel (I), in denen X für eine Hydroxy-Gruppe steht, können mit Aminen und Alkali- bzw. Erdalkalihydroxiden, -carbonaten oder -hydrogencarbonaten sowie Ammoniumhydroxidsalze bilden, außerdem können sie mit Schwermetallsalzen Metall-Komplexe bilden.

Schließlich wurde gefunden, daß die neuen E/Z-Isomerengemische und die reinen Z-Isomeren von $N^{\alpha}$-(2-Cyan-2-alkoximinoacetyl)-aminosäure-derivaten und -peptiden der allgemeinen Formel (I) sowie deren gegebenenfalls substituierte Ammonium-, Alkali- und Erdalkali-Salze sowie Metallsalz-Komplexe insbesondere starke fungizide Eigenschaften aufweisen. Dabei zeigen überraschenderweise die erfindungsgemäßen Verbindungen eine höhere Wirkung als die aus dem Stand der Technik bekannten Verbindungen, wie das Zinkethylen-1,2-bis-dithiocarbamat, welches eine wirkungsmäßig naheliegende Verbindung ist. Die erfindungsgemäßen Stoffe stellen somit eine Bereicherung der Technik dar.

Die erfindungsgemäßen E/Z-Isomerengemische und die reinen Z-Isomeren von $N\alpha$-(2-Cyan-2-alkoximinoacetyl)-aminosäurederivaten und -peptiden sind durch die allgemeine Formel (I) allgemein definiert. Besonders bevorzugt sind diejenigen Verbindungen der allgemeinen Formel (I), in denen

R für geradkettiges oder verzweigtes Alkyl mit 1 bis 4 Kohlenstoffatomen, Allyl, Propargyl, Cyanmethyl, Cyanethyl, für 1,2,4-Triazol-1-ylalkyl und Pyrazol-1-ylalkyl mit 1 bis 4 Kohlenstoffatomen im Alkylteil, für gegebenenfalls im Phenylteil einfach oder zweifach, gleich oder verschieden substituiertes Phenylalkyl mit 1 oder 2 Kohlenstoffatomen im Alkylteil steht, wobei als Substituenten insbesondere genannt seien: Fluor, Chlor, Cyano, Nitro, Hydroxy, Methyl, Methoxy, Methylthio, Methylsulfinyl, Methylsulfonyl und gegebenenfalls einfach oder zweifach, gleich oder verschieden durch Fluor oder Chlor substituiertes Phenyl; ferner für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht;

$R^1$ für Wasserstoff, Methyl, Ethyl sowie für jeweils gegebenenfalls einfach oder zweifach, gleich oder verschieden substituiertes Phenyl oder Benzyl steht, wobei als Substituenten insbesondere die bei R bereits genannten Phenylsubstituenten in Frage kommen;

$R^2$ für Wasserstoff, Methyl oder Ethyl steht;

$R^3$ für Wasserstoff, geradkettiges oder verzweigtes Alkyl mit 1 bis 4 Kohlenstoffatomen, Hydroxymethyl, 1-Hydroxyethyl, für Methoxycarbonylmethyl, Methoxycarbonylethyl, Ethoxycarbonylmethyl, Ethoxycarbonylethyl, Hydroxycarbonylmethyl, Hydroxycarbonylethyl, Aminocarbonylmethyl, Aminocarbonylethyl, für 1,2,4-Triazol-1-ylalkyl, 1,2,4-Triazol-4-ylalkyl, Imidazol-4-ylalkyl und Pyrazol-1-ylalkyl mit jeweils 1 bis 4 Kohlenstoffatomen im Alkylteil, für Cyanmethyl, Cyanethyl, für Allyl, für Propargyl, für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht; ferner für gegebenenfalls im Phenylteil einfach oder zweifach, gleich oder verschieden substituiertes Phenylalkyl mit 1 oder 2 Kohlenstoffatomen im Alkylteil oder einfach oder zweifach, gleich oder verschieden substituiertes Phenyl steht, wobei als Substituenten die bei R bereits genannten Phenylsubstituenten in Frage kommen, ferner für die Gruppierung $R^4$-$SO_n$-Z steht, wobei

$R^4$ für Wasserstoff, Methyl, Ethyl oder für gegebenenfalls im Phenylteil einfach oder zweifach, gleich oder verschieden substituiertes Phenylalkyl mit 1 oder 2 Kohlenstoffatomen im Alkylteil steht, wobei als Substituenten insbesondere die bei R bereits genannten Phenylsubstituenten in Frage kommen,
n für die Zahlen 0, 1 oder 2 steht und
Z für eine geradkettige oder verzweigte Alkylenkette mit 1 oder 2 Kohlenstoffatomen steht;

$R^1$ und $R^3$ gemeinsam mit dem Stickstoffatom und dem Kohlenstoffatom, an die sie gebunden sind, für einen 5- bis 6-gliedrigen Heterocyclus stehen;

$R^2$ und $R^3$ gemeinsam mit dem Kohlenstoffatom, an das sie gebunden sind, für Cyclopropyliden stehen, und

X für die Gruppierungen $OR^I$ oder $-NR^{II}R^{III}$ steht,
wobei

$R^I$ für Wasserstoff, Methyl, Ethyl, Allyl sowie Propargyl steht;

$R^{II}$ für Wasserstoff, Methyl oder Ethyl steht;

$R^{III}$ für Wasserstoff, Methyl, Ethyl, n-Propyl, i-Propyl, n-Butyl, i-Butyl, sec.-Butyl, tert.-Butyl, Allyl, Propargyl, für Halogenalkyl mit 1 oder 2 Kohlenstoffatomen und 1 bis 3 gleichen oder verschiedenen Halogenatomen, wie Fluor- und Chloratomen, für Alkoxyalkyl mit jeweils 1 oder 2 Kohlenstoffaotmen im Alkoxyteil und im Alkylteil, für Dialkylaminoalkyl mit 1 oder 2 Kohlenstoffatomen in den einzelnen Alkylteilen, für Alkoxycarbonylalkyl, Hydroxycarbonylalkyl, Aminocarbonylalkyl, Alkylaminocarbonylalkyl oder Dialkylaminocarbonylalkyl mit jeweils 1 oder 2 Kohlenstoffatomen in jedem Alkylteil, Cyanoalkyl; ferner für gegebenenfalls im Phenylteil einfach oder zweifach, gleich oder verschieden substituiertes Phenylalkyl mit 1 oder 2 Kohlenstoffatomen im Alkylteil, für einfach oder zweifach, gleich oder verschieden substituiertes Phenyl steht, wobei als Substituenten jeweils die bei R bereits genannten Phenylsubstituenten in Frage kommen sowie für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht;
$R^{II}$ und $R^{III}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann und gegebenenfalls durch Cyano, Fluor, Chlor, Brom, Iod, Methyl, Ethyl, i-Propyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl sowie Methylethylaminocarbonyl substituiert sein kann.

Bevorzugte erfindungsgemäße Verbindungen sind auch die für bestimmte Substituentendefinitionen möglichen Alkali-, Erdalkali- und gegebenenfalls substituierten Ammoniumsalze der E/Z-Isomerengemische und reinen Z-Isomeren der $N^{\alpha}$-(2-Cyan-2-alkoximinoacetyl)-aminosäure- und -peptid-Derivate der allgemeinen Formel (I).

Für diese Salzbildung können Alkali- und Erdalkalihydroxide, -carbonate oder -hydrogencarbonate oder Amine und Ammoniumhydroxid eingesetzt werden. Hierzu gehören vorzugsweise Natriumhydroxid, Natriumcarbonat, Natriumhydrogencarbonat, Kaliumhydroxid, Kaliumcarbonat, Calciumhydroxid, Bariumhydroxid; Ammoniak; primäre Amine, wie Methylamin und Isopropylamin; sekundäre Amine, wie Dimethylamin und Dicyclohexylamin; tertiäre Amine, wie Triethylamin, Pyridin und N,N-Dimethylbenzylamin sowie Ammoniumhydroxide, wie Benzyltrimethyl-ammoniumhydroxid.

Außerdem bevorzugte erfindungsgemäße Verbindungen sind Additionsprodukte aus Salze von Metallen der II. bis IV. Haupt- und der I. und II. sowie IV. bis VIII. Nebengruppe und denjenigen $N^{\alpha}$-(2-Cyan-2-alkoximinoacetyl)-aminosäure- und -peptid-Derivaten der allgemeinen Formel (I), in denen die Substituenten, R, $R^1$, $R^2$, $R^3$ and X die Bedeutungen haben, die bereits für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man beispielsweise Glycinbenzylamid-hydrobromid und (Z)-2-Cyan-2-methoximino-acetylchlorid als Ausgangsstoffe, so kann der Ablauf des erfindungsgemäßen Verfahrens durch das folgende Formelschema wiedergegeben werden:

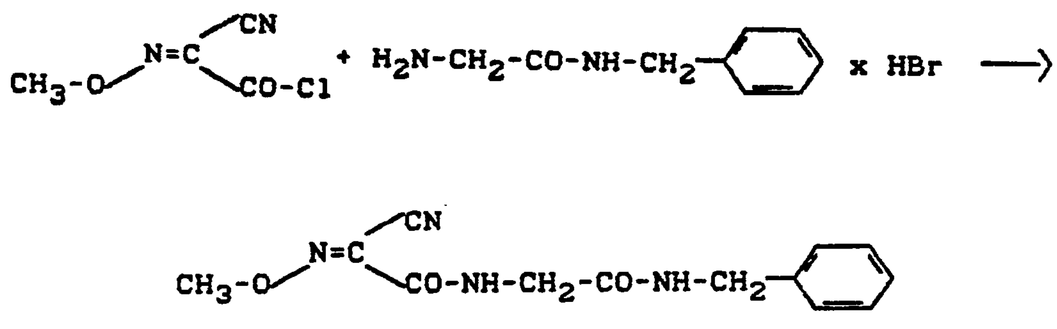

Die für die Durchführung des erfindungsgemäßen Verfahrens als Ausgangsstoffe zu verwendenden E/Z-Isomerengemische und reinen Z-Isomeren der 2-Cyan-2-oximino-essigsäurederivate sind durch die allgemeine Formel (II) allgemein definiert. In dieser Formel hat R die Bedeutung, die bereits im Zusammenhang mit der Beschreibung der erfindungsgemäßen Stoffe der allgemeinen Formel (I) für diesen Substituenten genannt wurde, Q steht vorzugsweise für Chlor oder Alkoxy mit 1-4 C-Atomen.

Die Säurechloride der allgemeinen Formel (II) sind noch nicht bekannt; die entsprechenden Ester sind teilweise bekannt (vgl. z.B. J. Antibiot. 37, 557 (1984)). Man erhält die Säurechloride beispielsweise, wenn man Verbindungen der allgemeinen Formel (IV)

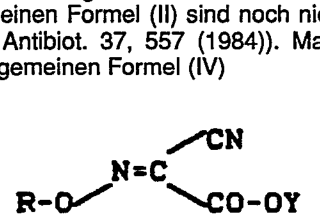

(IV)

in welcher
R die oben angegebene Bedeutung hat und

Y für Wasserstoff oder ein Alkalimetallatom wie beispielsweise Natrium oder Kalium steht,
mit einem Halogenierungsmittel gegebenenfalls in Gegenwart eines Verdünnungsmittels und gegebenenfalls in Gegenwart eines Katalysators umsetzt.

Als Verdünnungsmittel kommen für das obengenannte Verfahren zur Herstellung der Säurechloride der allgemeinen Formel (II) inerte organische Lösungsmittel in Frage. Hierzu gehören vorzugsweise Ether, wie Diethylether; chlorierte Kohlenwasserstoffe, wie Methylenchlorid; Ester, wie Essigester; Nitrile, wie Acetonitril; sowie Kohlenwasserstoffe, wie Toluol; Alkohole, wie Methanol oder i-Propanol; sowie Amide, wie Dimethylformamid.

Das Verfahren zur Herstellung der Säurechloride (II) wird mit einem Halogenierungsmittel durchgeführt. Man kann alle üblicherweise verwendbaren Halogenierungsmittel einsetzen. Vorzugsweise arbeitet man mit Oxalylchlorid, Phosphorpentachlorid, Thionylchlorid oder Phosgen. Dabei ist zu beachten, daß man nur bei Abwesenheit von freiem Chlorwasserstoff im Reaktionsgemisch die reinen Z-Isomeren erhält; andernfalls fallen unter teilweiser Isomerisierung E/Z-Isomerengemische an.

Das Verfahren zur Herstellung der Säurechloride (II) wird gegebenenfalls in Gegenwart eines Katalysators durchgeführt. Hierzu gehören vorzugsweise Dimethylformamid und Triphenylphosphin.

Die Reaktionstemperaturen können bei der Durchführung des Verfahrens zur Herstellung der Säurechloride der Formel (II) in einem größeren Bereich variiert werden. Im allgemeinen arbeitet man bei Temperaturen zwischen -20 °C und 80 °C, vorzugsweise bei Temperaturen zwischen -10 °C und 30 °C, insbesondere bei 0 °C.

Bei der Durchführung des Verfahrens zur Herstellung der Säurechloride (II) setzt man auf 1 Mol der Verbindung der Formel (II) im allgemeinen 1 bis 10 Mol, vorzugsweise 1 bis 3 Mol Halogenierungsmittel ein.
Zur Isolierung der Verbindung der allgemeinen Formel (I) wird das Reaktionsgemisch in üblicher Art und Weise aufgearbeitet.

Die Verbindungen der allgemeinen Formel (IV) sind bisher noch nicht bekannt. Sie lassen sich jedoch nach im Prinzip bekannten Verfahren in einfacher Weise herstellen. So erhält man beispielsweise die Verbindungen der allgemeinen Formel (IV), indem man die Carbonsäureester der allgemeinen Formel (V)

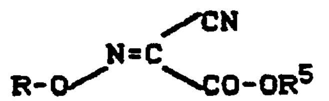

(V)

in welcher
R die oben angegebene Bedeutung hat und
$R^5$ für Methyl oder Ethyl steht,
in einem Lösungsmittel, wie beispielsweise Wasser, Methanol oder Ethanol mit einem Alkalihydroxid, wie beispielsweise Kalium- oder Natriumhydroxid verseift oder zunächst mit einem Alkalihydroxid, wie beispielsweise Natriumhydroxid, und anschließend mit einer Säure, wie beispielsweise Salzsäure oder Schwefelsäure, oder mit einem sauren Ionenaustauscher in Gegenwart eines Verdünnungsmittels, wie beispielsweise Wasser, Alkohole, Ether oder Gemische von Alkoholen oder Ethern mit Wasser, bei Temperaturen zwischen 0 °C und 80 °C, vorzugsweise zwischen 20 °C und 40 °C, umsetzt.

Die Carbonsäureester der allgemeinen Formel (V) sind teilweise bekannt (vergl. z. B. J. Antibiot. 37, 557 - 571 (1984)).

Sie können erhalten werden, indem man Amide der allgemeinen Formel (VI)

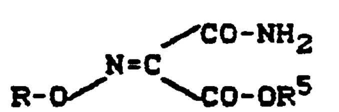

(VI)

in welcher
R und $R^5$ die oben angegebene Bedeutung haben,
mit einem Dehydratisierungsmittel, wie beispielsweise Trifluoressigsäureanhydrid, Trichloracetylchlorid, Mesylchlorid, Tosylchlorid, Titantetrachlorid oder N,N-Dimethylchlorformimidiumchlorid, in Gegenwart einer tertiären Base, wie beispielsweise Pyridin, Triethylamin oder N-Methylmorpholin, und gegebenenfalls in Gegenwart eines Lösungsmittels, wie beispielsweise Dioxan, Tetrahydrofuran, Acetonitril, Methylenchlorid oder Pyridin bei Temperaturen zwischen -20 °C und 60 °C dehydratisiert.

Die Amide der allgemeinen Formel (VI) sind teilweise bekannt (vergl. z. B. J. Antibiot. 37, 557 - 571 (1984)).

Sie können erhalten werden, indem man Verbindungen der allgemeinen Formel (VII)

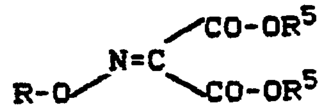

(VII)

in welcher
R und $R^5$ die oben angegebenen Bedeutungen haben,
mit Ammoniak in Gegenwart eines Lösungsmittels, wie beispielsweise Methanol, Ethanol, Diethylether, Dimethoxyethan, Dimethylformamid oder Acetonitril bei Temperaturen zwischen -20 °C und 30 °C umsetzt.

Die Verbindungen der allgemeinen Form (VII) sind teilweise bekannt (vergl. z. B. J. Antibiot. 37, 557 - 571 (1984)).

Sie können erhalten werden, indem man Verbindungen der allgemeinen Formel (VIII)

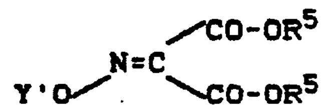

(VIII)

in welcher
$R^5$ die oben angegebene Bedeutung hat und
Y' für Wasserstoff oder für ein Alkalimetall, wie beispielsweise Natrium oder Kalium steht,
mit Verbindungen der allgemeinen Formel (IX)
R-W (IX)
in welcher
R die oben angegebene Bedeutung hat und
W für Halogen oder einen Sulfonyl(di)oxy-Rest wie vorzugsweise Chlor, Brom, Iod, $OSO_2OCH_3$, $OSO_2OC_2H_5$, $-OSO_2CH_3$ oder

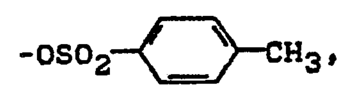

steht,
gegebenenfalls in Gegenwart einer Base, wie beispielsweise Kaliumcarbonat, Triethylamin oder Diazabicycloundecan (DBU), und in Gegenwart eines Lösungsmittels, wie beispielsweise Aceton, Dimethylformamid, Dimethylsulfoxid oder Acetonitril, bei Temperaturen zwischen 0 °C und 120 °C umsetzt.

Die Verbindungen der allgemeinen Formeln (VIII) und (IX) sind allgemein bekannte Verbindungen der organischen Chemie.

Die außerdem für die Durchführung des erfindungsgemäßen Verfahrens als Ausgangsstoffe zu verwendenden Aminosäure-Derivate sind durch die allgemeine Formel (III) allgemein definiert. In dieser Formel haben $R^1$, $R^2$, $R^3$ und X die Bedeutungen, die bereits im Zusammenhang mit der Beschreibung der erfindungsgemäßen Stoffe der Formel (I) für diese Substituenten genannt wurden.

Die Aminosäure-Derivate der allgemeinen Formel (III) sind teilweise bekannt (vergl. z. B. Houben-Weyl, Methoden der organischen Chemie, Band XV, Teil 1 und 2, Georg Thieme Verlag, Stuttgart 1974; bzw. R.C. Sheppard, A Specialist Periodical Report, Amino-acids, Peptides and Proteins, The Royal Society of Chemistry, Burlington House, London 1978, bzw. I.P. Greenstein and M. Winitz, Chemistry of Amino Acids, I. Wiley Sons Inc., New York, London 1961; bzw. E. Schröder and K. Lübke, The Peptides Vol. I. Academic Press, New York, London 1965) oder können nach den dort angegebenen Verfahren erhalten werden.

Als Verdünnungsmittel kommen für das erfindungsgemäße Verfahren inerte organische Lösungsmittel in Frage. Hierzu gehören Ketone, wie Aceton oder Ethylmethylketon; Ester wie Ethyl- oder Methylacetat; Amide wie Dimethylformamid; Nitrile wie Acetonitril; Chlorkohlenwasserstoffe, wie Methylenchlorid oder Chloroform ; Kohlenwasserstoffe, wie Toluol; oder Ether, wie Tetrahydrofuran; bzw. deren Mischungen.

Als Säurebindemittel kommen für das erfindungsgemäße Verfahren übliche anorganische und organische Säurebinder in Frage. Hierzu gehören vorzugsweise tertiäre Amine, wie Triethylamin, Pyridin oder N-Methylmorpholin; sowie anorganische Basen, wie Natriumcarbonat oder Calciumcarbonat.

Das erfindungsgemäße Verfahren wird gegebenenfalls in Gegenwart eines Katalysators durchgeführt. Beispielsweise genannt seien 4-Dimethylaminopyridin, 1-Hydroxy-benzotriazol oder Dimethylformamid.

Die Temperaturen können bei der Durchführung des Verfah rens in einem größeren Bereich variiert werden. Im allgemeinen arbeitet man zwischen -60 °C bis +120 °C, vorzugsweise bei -20 °C bis +40 °C.

Die erfindungsgemäßen Verbindungen der allgemeinen Formel (I) fallen je nach eingesetztem Ausgangsprodukt und je nach Reaktionsbedingungen als E/Z-Isomerengemische oder als reine Z-Isomere an. Dabei wird die E/Z-Isomereisierung durch die Anwesenheit von freiem Chlorwasserstoff begünstigt.

Bei der Durchführung des erfindingsgemäßen Verfahrens arbeitet man vorzugsweise in äquimolaren Mengen, wobei die Base je nach gewünschtem Reaktionsprodukt; (E/Z-Isomerengemisch oder reines Z-Isomeres) in Unter- oder Überschuß eingesetzt wird.

Die Aminosäurederivate der allgemeinen Formel (III) werden als reine Enantiomere (D bzw. L-Form) oder als Racemate eingesetzt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Schädlingsbekämpfungsmittel geeignet.

Zum Beispiel werden fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola; Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Schädlingskbekämpfungsmittel können die erfindungsgemäßen Wirkstoffe mit besonders gutem Erfolg zur Bekämpfung von Phytophthora-Arten, wie beispielsweise Phytophthora infestans, an Tomaten eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normal

druck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farb stoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akarizidin, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.BV. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

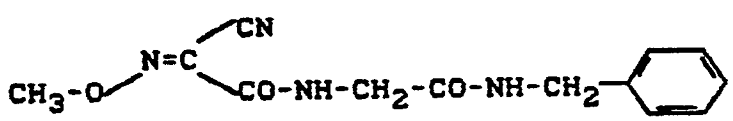

(Z-Isomer)

Zu einer Lösung von 12,3 g (0,05 Mol) Glycinbenzylamid-hydrobromid in 100 ml trockenem Dimethylformamid gibt man 12,2 g (0,12 Mol) Triethylamin und bei 0 °C tropfenweise 7,4 g (0,05 Mol) (Z)-2-Cyan-2-methoximino-acetylchlorid und rührt das Reaktionsgemisch 1 Stunde bei 0 °C und 17 Stunden bei Raumtemperatur. Das Lösungsmittel wird im Vakuum abdestilliert, der Rückstand in 250 ml Dichlormethan gelöst, die Lösung mit 100 ml 1M Salzsäure, 100 ml gesättigter Natriumhydrogencarbonatlösung und 150 ml Wasser gewaschen, über Natriumsulfat getrocknet und im Vakuum eingedampft. Der Rückstand wird aus Toluol/Essigester/Ligroin (1:1:1) umkristallisiert.

Man erhält 7,35 g (53,6 % der Theorie) Nα-[(Z)-2-Cyan-2-methoximino-acetyl]-glycinbenzylamid vom Schmelzpunkt 120 °C - 121 °C.

Herstellung der Ausgangsprodukte

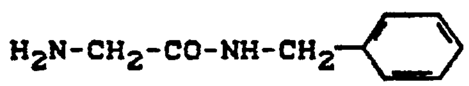 x HBr

In eine Lösung von 250 g (1,2 Mol) N-Benzyloxycarbonylglycin und 120,7 g (1,2 Mol) Triethylamin in 1500 ml trockenem Tetrahydrofuran tropft man bei -50 °C 165,6 g (1,2 Mol) Chlorameisensäureisobutylester und rührt 10 Minuten bei -50 °C. Dann gibt man in einem Guß eine vorgekühlte Lösung von 140,8 g (1,32 Mol) Benzylamin in 200 ml trockenem Tetrahydrofuran hinzu, rührt 30 Minuten bei - 15 °C und 15 Stunden bei Raumtemperatur. Das Reaktionsgemisch wird filtriert, das Filtrat im Vakuum eingedampft, der Rückstand in 3 l Chloroform gelöst, die Lösung mit Wasser, 1M Salzsäure, gesättigter Natriumhydrogencarbonatlösung und Wasser gewaschen, über Natriumsulfat getrocknet und im Vakuum eingedampft. Der Rückstand wird mit 300 ml Petrolether 30 Minuten verrührt, die farblosen Kristalle abgesaugt und getrocknet.

Man erhält 169 g (47 % der Theorie) $N^{\alpha}$-Benzyloxycarbonylglycinbenzylamid vom Schmelzpunkt 114 °C - 117 °C.

22,4 g (0,075 Mol) des so erhaltenen $N^{\alpha}$-Benzyloxycarbonylglycinbenzylamid übergießt man bei Raumtemperatur mit 82 ml einer 33-prozentigen Lösung von Bromwasserstoff in Eisessig und rührt die Suspension 2 Stunden bei Raumtemperatur. Nach Verdünnen mit 140 ml Ether werden die farblosen Kristalle abgesaugt, mit Ether gewaschen und im Vakuum über Kaliumhyroxid getrocknet.

Man erhält 18 g (97,9 % der Theorie) Glycin-benzylamid-hydrobromid vom Schmelzpunkt 187°C–188°C.

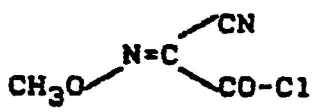

Man suspendiert 23,5 g (0,14 Mol) gut getrocknetes Kalium-(Z)-2-cyan-2-methoximino-acetat in 350 ml absolutem Ether, gibt einige Tropfen absolutes Dimethylformamid hinzu, tropft bei 0 °C in 35 Minuten 53,4 g (0,42 Mol) Oxalylchlorid (zuvor mit 2 g Kaliumcarbonat 1 Stunde verrührt) zu und rührt 2 Stunden bei 0 °C. Das Reaktionsgemisch wird filtriert, das Filtrat im Vakuum eingedampft und der Rückstand zweimal mit trockenem Dichlormethan im Vakuum abgedampft.

Man erhält 15,7 g (76 % der Theorie) (Z)-2-Cyan-2-methoximino-acetylchlorid als orangefarbenes Öl, das sofort weiter umgesetzt wird.

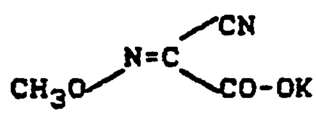

In eine Lösung von 34,3 g (0,2 Mol) (Z)-2-Cyan-2-methoximino-essigsäureethylester (91-prozentig) in 200 ml Ethanol tropft man eine Lösung von 11,8 g (0,21 Mol) Kaliumhydroxid in 80 ml Wasser und rührt 1,5 Stunden bei 40 °C. Die Reaktionslösung wird im Vakuum bei 40 °C eingedampft, der Rückstand mit Ethanol und Ether gewaschen und im Vakuum bei Raumtemperatur getrocknet.

Man erhält 28,3 g (85 % der Theorie) Kalium-(Z)-2-cyan-2-methoximino-acetat als beigefarbenes Pulver, das sich bei 111 °C (Peak-Temperatur der Differentialthermoanalyse) explosionsartig zersetzt. Die Z-Konfiguration ist durch $^{13}C$-NMR sichergestellt.

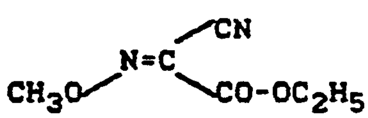

In eine Lösung von 33,5 g (0,177 Mol) 92-prozentigem (Z)-2-Methoximino-malonsäure-monoamid-ethylester und 28,0 g (0,35 Mol) Pyridin in 260 ml trockenem Dioxan tropft man bei 0 °C in 25 Minuten 71,4 g (0,336 Mol) Trifluoressigsäureanhydrid (99-prozentig) und rührt 2 Stunden bei Raumtemperatur. Das Reaktionsgemisch wird mit 260 ml Dichlormethan verdünnt, zweimal mit je 200 ml Wasser, mit 150 ml

10-prozentiger Natrium hydrogencarbonatlösung und 200 ml Wasser gewaschen, über Natriumsulfat getrocknet und im Vakuum eingedampft.

Man erhält in einer Reinheit von 88 % (GC) 23,4 g (74 % der Theorie) (Z)-2-Cyan-2-methoximinoessigsäureethylester als rotbraunes Öl vom Brechungsindex $n_D^{20}$ = 1,4399.

Die Z-Konfiguration ist durch $^{13}$C-NMR sichergestellt.

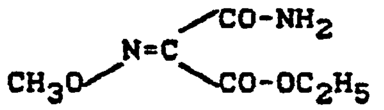

In eine Lösung von 44,2 g (0,196 Mol) 90-prozentigem 2-Methoximino-malonsäurediethylester in 200 ml absolutem Ethanol tropft man bei Raumtemperatur 115 ml (0,587 Mol) einer 5,1 M Lösung von Ammoniak in Methanol und rührt 24 Stunden bei Raumtemperatur.

Nach Eindampfen im Vakuum hinterbleiben in einer Reinheit von 92 % (nach Gaschromatographie) 36,8 g (99 % der Theorie) (Z)-2-Methoximinomalonsäuremonoamid-ethylester als gelbbraunes Öl vom Brechungsindex $n_D^{20}$= 1,4716.

Die Z-Konfiguration wurde durch $^{13}$C-NMR sichergestellt.

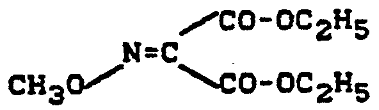

Zu einer Lösung von 108,9 g (0,5 Mol) 87-prozentigem 2-Hydroximino-malonsäure-diethylester in 400 ml Dimethylsulfoxid gibt man 138 g (1 Mol) Kaliumcarbonat, tropft in 20 Minuten 81,2 g (0,625 Mol) Dimethylsulfat zu, wobei die Temperatur auf 70 °C ansteigt, und rührt 3 Stunden bei 60 °C. Nach Abkühlung wird filtriert, das Filtrat in 1 l Wasser gegossen und dreimal mit je 600 ml Toluol/Essigester (5:1) extrahiert. Der mit 600 ml Wasser gewaschen Extrakt wird über Natriumsulfat getrocknet und im Vakuum eingedampft.

In einer Reinheit von 95 % (nach Gaschromatographie) erhält man 73,9 g (69 % der Theorie) 2-Methoximino-malonsäure-diethylester als orangefarbenes Öl vom Brechungsindex $n_D^{20}$= 1,4400.

<u>Beispiel 2</u>

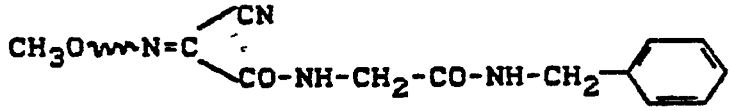

(E/Z-Isomerengemisch)

Zu einer Lösung von 5,36 g (0,218 Mol) Glycinbenzylamidhydrobromid in 60 ml absolutem Dimethylformamid gibt man 4,41 g (0,437 Mol) Triethylamin und bei 0 °C tropfenweise 3,2 g (0,218 Mol) 2-Cyan-2-methoximinoacetylchlorid. Man rührt 1 Stunde bei 0 °C und 15 Stunden bei Raumtemperatur. Das Lösungsmittel wird im Vakuum abdestilliert, der Rückstand in 100 ml Dichlormethan gelöst, die Lösung mit je 30 ml 1M Salzsäure, 10-prozentiger Natriumhydrogencarbonat-Lösung und Wasser gewaschen, über Natriumsulfat getrocknet und eingedampft. Der Rückstand wird mit Ether verrieben.

Man erhält 4,41 g (73,8 % der Theorie) N$^{\alpha}$-(2-Cyan-2-methoximino-acetyl)-glycinbenzylamid vom Schmelzpunkt 105 °C - 106 °C.

Nach $^{13}$C-NMR (DMSO) handelt es sich um ein E/Z-Gemisch (ca. 1:1).

<u>Herstellung des Ausgangsproduktes</u>

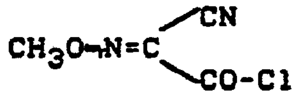

Zu einer Suspension von 25,0 g (0,15 Mol) Kalium-(Z)-2-cyan-2-methoximino-acetat in 400 ml absolutem Ether gibt man einige Tropfen Dimethylformamid, tropft bei 0 °C in 30 Minuten 57,2 g (0,45 Mol) handelsübliches Oxalylchlorid zu und rührt 2 Stunden bei 0 °C. Das Reaktionsgemisch wird filtriert, das Filtrat im Vakuum eingedampft und zur Entfernung von überschüssigem Oxalylchlorid noch einmal mit 100 ml Dichlormethan im Vakuum abgedampft.

Man erhält 16,8 g 2-Cyan-2-methoximino-acetylchlorid als orangefarbenes Öl, das sofort weiter umgesetzt wird.

Beispiel 3

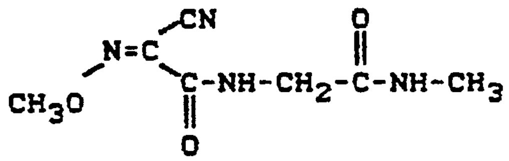

Eine Lösung von 53 g (0,34 mol) (Z)-2-Cyan-2-methoximino-essigsäureethylester und 33 g (0,375 mol) Glycinmethylamid in 300 ml Methanol wird 15 Stunden bei Raumtemperatur gerührt. Man saugt den kristallinen Niederschlag ab, wäscht ihn mit Petrolether und trocknet ihn. Man erhält 48 g (71,2 % der Theorie) Nα-[(Z)-2-Cyan-2-methoximinoacetyl]glycinmethylamid vom Schmelzpunkt 118° C.

Herstellung der Ausgangsverbindung:

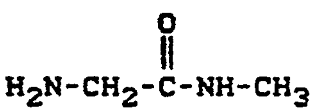

In eine Suspension von 417 g (3 mol) Glycinethylester-hydrochlorid in 1600 ml Methanol leitet man bei 0° C 930 g (30 mol) Methylamin und rührt 15 Stunden bei Raumtemperatur. Dann gibt man 630 g (3 mol) 8,6-prozentige Natriummethylatlösung hinzu, filtriert nach 15 Minuten über Kieselgur und dampft ein. Man erhält 238 g (89 % der Theorie) Glycinmethylamid als hellgelbes Öl mit einem Gehalt von 99 % (GC).

In analoger Weise und entsprechend dem erfindungsgemäßen Verfahren werden die nachfolgenden E/Z-Isomerengemische und reinen Z-Isomeren der Verbindungen der allgemeinen Formel (I)

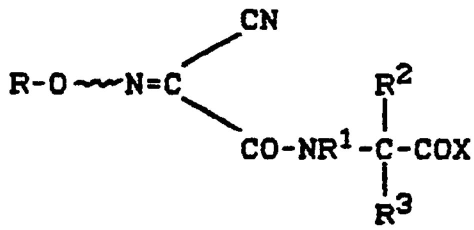

(I)

erhalten:

| Beisp.-Nr. | R | $R^1$ | $R^2$ | $R^3$ | X | Physikalische Konstante |
|---|---|---|---|---|---|---|
| 4 | $-CH_3$ | H | H | H | $-OC_2H_5$ | Fp: 69-71° C (Z-Isomer) |
| 5 | $-CH_3$ | H | H | H | $-N(C_2H_5)_2$ | Fp: 89-90° C (E/Z-Isomerengemisch 1:1) |
| 6 | $-CH_3$ | H | H | H | $-NH-CH_3$ | Fp:104-106° C (E/Z-Isomerengemisch 1:1) |
| 7 | $-CH_3$ | H | H | H | $-N(C_2H_5)_2$ | Fp:107-108° C (Z-Isomer) |

Verwendungsbeispiel

In dem nachfolgenden Verwendungsbeispiel wird die nachfolgend angegebene Substanz als Vergleichsverbindung eingesetzt:

(A)

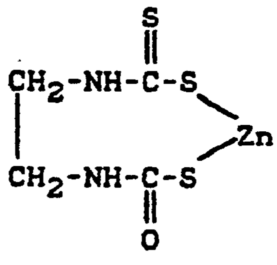

Beispiel A

Phytophthora-Test (Tomate) /protektiv

Lösungsmittel:4,7 Gewichtsteile Aceton
Emulgator; 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z. B. die Verbindungen der Herstellungsbeispiele: 2, 3, 5 und 6.

**Patentansprüche**

1. E/Z-Isomerengemische und reine Z-Isomere von $N^{\alpha}$-(2-Cyan-2-alkoximino-acetyl)-aminosäurederivaten und -peptiden der allgemeinen Formel (I)

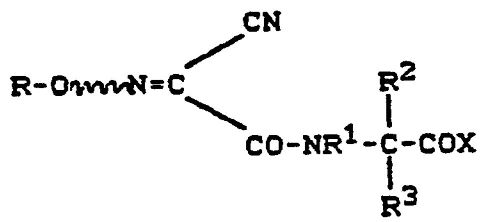

(I)

in welcher
R für geradkettiges oder verzweigtes Alkyl mit 1 bis 8 Kohlenstoffatomen, Alkenyl oder Alkinyl mit jeweils 2 bis 4 Kohlenstoffatomen, für Cyanalkyl mit 1 bis 4 Kohlenstoffatomen, für 1,2,4-Triazol-1-ylalkyl und Pyrazol-1-ylalkyl mit jeweils 1 bis 6 Kohlenstoffatomen im Alkylteil, für gegebenenfalls im Phenylteil einfach bis dreifach, gleich oder verschieden substituiertes Phenylalkyl mit 1 bis 4 Kohlenstoffatomen im Alkylteil steht, wobei als Substituenten vorzugsweise genannt seien:
Halogen, Cyano, Nitro, Hydroxy, Alkyl, Alkoxy, Alkylsulfinyl, Alkylsulfonyl und Alkylthio mit jeweils 1 bis 4 Kohlenstoffatomen und gegebenenfalls einfach bis dreifach, gleich oder verschieden durch halogen substituiertes Phenyl; oder für gegebenenfalls einfach bis dreifach, gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen genannt seien;
$R^1$ für Wasserstoff, geradkettiges oder verzweigtes Alkyl mit 1 bis 4 Kohlenstoffatomen oder für jeweils gegebenenfalls einfach bis dreifach, gleich oder verschieden substituiertes Phenyl- oder Benzyl steht, wobei als Substituenten vorzugsweise die bei R bereits genannten Phenylsubstituenten in Frage kommen;
$R^2$ für Wasserstoff oder geradkettiges oder verzweigtes Alkyl mit 1 bis 4 Kohlenstoffatomen steht;
$R^3$ für Wasserstoff, geradkettiges oder verzweigtes Alkyl mit 1 bis 6 Kohlenstoffatomen, für geradkettiges oder verzweigtes Hydroxyalkyl mit 1 bis 4 Kohlenstoffatomen, für Alkoxycarbonylalkyl mit jeweils 1 bis 4 Kohlenstoffatomen im Alkoxyteil und im Alkylteil, für Hydroxycarbonylalkyl und Aminocarbonylalkyl mit jeweils 1 bis 4 Kohlenstoffatomen im Alkylteil, für 1,2,4-Triazol-1-ylalkyl, 1,2,4-Triazol-4-ylalkyl, Imidazol-4-ylalkyl und Pyrazol-1-ylalkyl mit jeweils 1 bis 6 Kohlenstoffatomen im Alkylteil, für Cyanalkyl mit 1 bis 4 Kohlenstoffatomen, für Alkenyl oder Alkinyl mit jeweils 2 bis 4 Kohlenstoffatomen, für gegebenenfalls einfach bis dreifach, gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen genannt seien, ferner für gegebenenfalls im Phenylteil einfach bis dreifach, gleich oder verschieden substituiertes Phenyl oder Phenylalkyl mit 1 bis 4 Kohlenstoffatomen im Alkylteil steht, wobei als Substituenten jeweils vorzugsweise die bei R bereits genannten Phenylsubstituenten in Frage kommen, oder für die Gruppierung $R^4$–$SO_n$–Z– steht, wobei
$R^4$ für Wasserstoff, geradkettiges oder verzweigtes Alkyl mit 1 bis 4 Kohlenstoffatomen, für gegebenenfalls im Phenylteil einfach bis dreifach, gleich oder verschieden substituiertes Phenylalkyl mit 1 bis 4 Kohlenstoffatomen im Alkylteil steht, wobei als Substituenten die bei R bereits genannten Phenylsubstituenten in Frage kommen;
n für die Zahlen 0, 1 oder 2 steht und
Z für eine geradkettige oder verzweigte Alkylenkette mit 1 bis 4 Kohlenstoffatomen steht;
$R^1$ und $R^3$ gemeinsam mit dem Stickstoffatom und dem Kohlenstoffatom, an die sie gebunden sind, für einen 5- oder 6-gliedrigen Heterocyclus stehen;

$R^2$ und $R^3$ gemeinsam mit dem Kohlenstoffatom, an das sie gebunden sind, für Cycloalkyliden mit 3 bis 6 Kohlenstoffatomen stehen;
X für die Gruppierungen $-OR^I$ oder $-NR^{II}R^{III}$ steht, wobei
$R^I$ für Wasserstoff, geradkettiges oder verzweigtes Alkyl mit 1 bis 4 Kohlenstoffatomen, Alkenyl oder Alkinyl mit jeweils 2 bis 4 Kohlenstoffatomen steht;
$R^{II}$ für Wasserstoff oder geradkettiges oder verzweigtes Alkyl mit 1 bis 4 Kohlenstoffatomen steht;
$R^{III}$ für Wasserstoff, geradkettiges oder verzweigtes Alkyl mit 1 bis 4 Kohlenstoffatomen, für Alkenyl oder Alkinyl mit jeweils 2 bis 4 Kohlenstoffatomen, für Halogenalkyl mit 1 bis 4 Kohlenstoffatomen und 1 bis 5 gleichen oder verschiedenen Halogenatomen, für Alkoxyalkyl mit jeweils 1 bis 4 Kohlenstoffatomen im Alkoxyteil und im Alkylteil, für Dialkylaminoalkyl mit bis zu 4 Kohlenstoffatomen in den einzelnen Alkylteilen, für Alkoxycarbonylalkyl mit jeweils 1 bis 4 Kohlenstoffatomen im Alkoxyteil und im Alkylteil, Hydroxycarbonylalkyl mit 1 bis 4 Kohlenstoffatomen im Alkylteil, Aminocarbonylalkyl, Alkylaminocarbonylalkyl oder Dialkylaminocarbonylalkyl mit jeweils 1 bis 4 Kohlenstoffatomen in jedem Alkylteil für Cyanalkyl mit 1 bis 4 Kohlenstoffatomen im Alkylteil, gegebenenfalls im Phenylteil einfach bis dreifach, gleich oder verschieden substituiertes Phenylalkyl mit 1 bis 4 Kohlenstoffatomen im Alkylteil oder einfach bis dreifach, gleich oder verschieden substituiertes Phenyl steht, wobei als Substituenten jeweils die bei R bereits genannten Phenylsubstituenten in Frage kommen sowie für gegebenenfalls gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen in Frage kommen; oder
$R^{II}$ und $R^{III}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann und gegebenenfalls durch Cyano, Halogen, Alkyl mit 1 bis 4 Kohlenstoffatomen, Hydroxycarbonyl, Alkoxycarbonyl mit 1 bis 4 Kohlenstoffatomen im Alkoxyteil, Aminocarbonyl sowie Alkylaminocarbonyl und Dialkylaminocarbonyl mit jeweils 1 bis 4 Kohlenstoffatomen in den einzelnen Alkylteilen substituiert sein kann,
ausgenommen die Verbindungen N-(Methoxycarbonylmethyl)-(2-cyano-2-methoximino)-acetamid und N-(Carbamoylmethyl)-(2-cyano-2-methoximino)-acetamid.

2. Verbindungen der allgmeinen Formel (I) gemäß Anspruch 1, worin
R für geradkettiges oder verzweigtes Alkyl mit 1 bis 4 Kohlenstoffatomen, Allyl, Propargyl, Cyanmethyl, Cyanethyl, für 1,2,4-Triazol-1-ylalkyl und Pyrazol-1-ylalkyl mit 1 bis 4 Kohlenstoffatomen im Alkylteil, für gegebenenfalls im Phenylteil einfach oder zweifach, gleich oder verschieden substituiertes Phenylalkyl mit 1 oder 2 Kohlenstoffatomen im Alkylteil steht, wobei als Substituenten insbesondere genannt seien: Fluor, Chlor, Cyano, Nitro, Hydroxy, Methyl, Methoxy, Methylthio, Methylsulfinyl, Methylsulfonyl und gegebenenfalls einfach oder zweifach, gleich oder verschieden durch Fluor oder Chlor substituiertes Phenyl; ferner für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht;
$R^1$ für Wasserstoff, Methyl, Ethyl sowie für jeweils gegebenenfalls einfach oder zweifach, gleich oder verschieden substituiertes Phenyl oder Benzyl steht, wobei als Substituenten insbesondere die bei R bereits genannten Phenylsubstituenten in Frage kommen;
$R^2$ für Wasserstoff, Methyl oder Ethyl steht;
$R^3$ für Wasserstoff, geradkettiges oder verzweigtes Alkyl mit 1 bis 4 Kohlenstoffatomen, Hydroxymethyl, 1-Hydroxyethyl, für Methoxycarbonylmethyl, Methoxycarbonylethyl, Ethoxycarbonylmethyl, Ethoxycarbonylethyl, Hydroxycarbonylmethyl, Hydroxycarbonylethyl, Aminocarbonylmethyl, Aminocarbonylethyl, für 1,2,4-Triazol-1-ylalkyl, 1,2,4-Triazol-4-ylalkyl, Imidazol-4-ylalkyl und Pyrazol-1-ylalkyl mit jeweils 1 bis 4 Kohlenstoffatomen im Alkylteil, für Cyanmethyl, Cyanethyl, für Allyl, für Propargyl, für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht; ferner für gegebenenfalls im Phenylteil einfach oder zweifach, gleich oder verschieden substituiertes Phenylalkyl mit 1 oder 2 Kohlenstoffatomen im Alkylteil oder einfach oder zweifach, gleich oder verschieden substituiertes Phenyl steht, wobei als Substituenten die bei R bereits genannten Phenylsubstituenten in Frage kommen, ferner für die Gruppierung $R^4{-}SO_n{-}Z$ steht, wobei
$R^4$ für Wasserstoff, Methyl, Ethyl oder für gegebenenfalls im Phenylteil einfach oder zweifach, gleich oder verschieden substituiertes Phenylalkyl mit 1 oder 2 Kohlenstoffatomen im Alkylteil steht, wobei als Substituenten insbesondere die bei R bereits genannten Phenylsubstituenten in Frage kommen,
n für die Zahlen 0, 1 oder 2 steht und
Z für eine geradkettige oder verzweigte Alkylenkette mit 1 oder 2 Kohlenstoffatomen steht;
$R^1$ und $R^3$ gemeinsam mit dem Stickstoffatom und dem Kohlenstoffatom, an die sie gebunden sind, für einen 5- bis 6-gliedrigen Heterocyclus stehen;
$R^2$ und $R^3$ gemeinsam mit dem Kohlenstoffatom, und das sie gebunden sind, für Cyclopropyliden stehen, und
X für die Gruppierungen $-OR^I$ oder $-NR^{II}R^{III}$ steht,
wobei
$R^I$ für Wasserstoff, Methyl, Ethyl, Allyl sowie Propargyl steht;

$R^{II}$ für Wasserstoff, Methyl oder Ethyl steht;
$R^{III}$ für Wasserstoff, Methyl, Ethyl, n-Propyl, i-Propyl, n-Butyl, i-Butyl, sec.-Butyl, tert.-Butyl, Allyl, Propargyl, für Halogenalkyl mit 1 oder 2 Kohlenstoffatomen und 1 bis 3 gleichen oder verschiedenen Halogenatomen, wie Fluor- und Chloratomen, für Alkoxyalkyl mit jeweils 1 oder 2 Kohlenstoffatomen im Alkoxyteil und im Alkylteil, für Dialkylaminoalkyl mit 1 oder 2 Kohlenstoffatomen in den einzelnen Alkylteilen, für Alkoxycarbonylalkyl, Hydroxycarbonylalkyl, Aminocarbonylalkyl, Alkylaminocarbonylalkyl oder Dialkylaminocarbonylalkyl mit jeweils 1 oder 2 Kohlenstoffatomen in jedem Alkylteil, Cyanoalkyl; ferner für gegebenenfalls im Phenylteil einfach oder zweifach, gleich oder verschieden substituiertes Phenylalkyl mit 1 oder 2 Kohlenstoffatomen im Alkylteil, für einfach oder zweifach, gleich oder verschieden substituiertes Phenyl steht, wobei als Substituenten jeweils die bei R bereits genannten Phenylsubstituenten in Frage kommen sowie für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht;
$R^{II}$ und $R^{III}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann und gegebenenfalls durch Cyano, Fluor, Chlor, Brom, Iod, Methyl, Ethyl, i-Propyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl sowie Methylethylaminocarbonyl substituiert sein kann.

3. Verfahren zur Herstellung von E/Z-Isomerengemischen und reinen Z-Isomeren von $N^{\alpha}$-(2-Cyan-2-alkoximinoacetyl)-aminosäurederivaten und -peptiden der Formel (I)

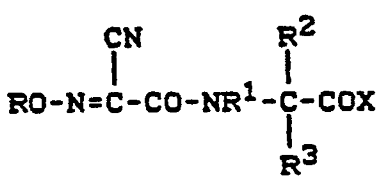

(I)

in welcher
R, $R^1$, $R^2$, $R^3$ und X die in Anspruch 1 angegebene Bedeutung haben,
ausgenommen die Verbindungen N-(Methoxycarbonylmethyl)-(2-cyano-2-methoximino)-acetamid und N-(Carbamoylmethyl)-(2-cyano-2-methoximino)-acetamid, dadurch gekennzeichnet, daß man die E/Z-Isomerengemische oder die reinen Z-Isomeren der 2-Cyan-2-oximino-essigsäure-derivate der allgemeinen Formel (II)

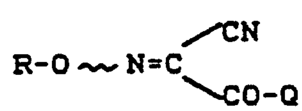

(II)

in welcher
R die oben angegebene Bedeutung hat und
Q für Chlor oder Alkoxy mit 1–4 C-Atomen steht,
mit Aminosäure-Derivaten der allgemeinen Formel (III)

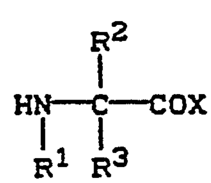

(III)

in welcher
$R^1$, $R^2$, $R^3$ und X die oben angegebenen Bedeutungen haben,
gegebenenfalls in Gegenwart eines Katalysators, gegebenenfalls in Gegenwart eines Säurebindemittels und gegebenenfalls in Gegenwart eines Verdünnungsmittels umsetzt.

4. Fungizide Mittel, gekennzeichnet durch einen Gehalt an mindestens einer Verbindung der allgemeinen Formel (I) gemäß Anspruch 1, ausgenommen die Verbindungen N-(Methoxycarbonylmethyl)-(2-cyano-2-methoximino)-acetamid und N-(Carbamoylmethyl)-(2-cyano-2-methoximino)-acetamid.

5. Verfahren zur Bekämpfung von pilzlichen Schädlingen, dadurch gekennzeichnet, daß man mindestens eine Verbindung der Formel (I) gemäß Anspruch 1 auf pilzliche Schädlinge und/oder ihren Lebensraum einwirken läßt, ausgenommen die Verbindungen N-(Methoxycarbonylmethyl)-(2-cyano-2-methoximino)-acetamid und N-(Carbamoylmethyl)-(2-cyano-2-methoximino)-acetamid.

6. Verwendung von Verbindungen der allgemeinen Formel (I) gemäß Anspruch 1 zur Bekämpfung von pilzlichen Schädlingen, ausgenommen die Verbindungen N-(Methoxycarbonylmethyl)-(2-cyano-2-methoximino)-acetamid und N-(Carbamoylmethyl)-(2-cyano-2-methoximino)-acetamid.

7. Verfahren zur Herstellung von fungiziden Mitteln, dadurch gekennzeichnet, daß man Verbindungen der allgemeinen Formel (I) gemäß Anspruch 1 mit Streckmitteln und/oder oberflächenaktiven Mitteln vermischt, ausgenommen die Verbindungen N-(Methoxycarbonylmethyl)-(2-cyano-2-methoximino)-acetamid und N-(Carbamoylmethyl)-(2-cyano-2-methoximino)-acetamid.

**Claims**

1. Mixtures of E/Z isomers and pure Z isomers of Nα-(2-cyano-2-alkoximino-acetyl)-amino acid derivatives and peptides of the general formula (I)

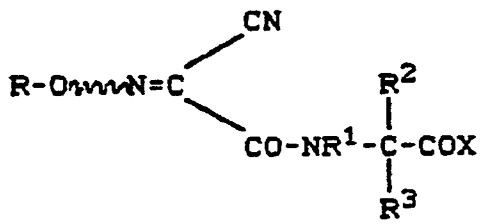

(I)

in which

R represents straight-chain or branched alkyl having 1 to 8 carbon atoms, alkenyl or alkinyl in each case having 2 to 4 carbon atoms, cyanoalkyl having 1 to 4 carbon atoms, 1,2,4-triazol-1-ylalkyl and pyrazol-1-ylalkyl in each case having 1 to 6 carbon atoms in the alkyl part, phenylalkyl, having 1 to 4 carbon atoms in the alkyl part, which is optionally mono- to trisubstituted in the phenyl part by identical or different substituents, substituents which may preferably be mentioned being: halogen, cyano, nitro, hydroxyl, alkyl, alkoxy, alkylsulphinyl, alkylsulphonyl and alkylthio in each case having 1 to 4 carbon atoms, and phenyl which is optionally mono- to trisubstituted by identical or different halogen substituents; or represents cycloalkyl, having 3 to 7 carbon atoms, which is optionally mono- to trisubstituted by identical or different substituents, substituents which may preferably be mentioned being halogen and alkyl having 1 to 4 carbon atoms;

$R^1$ represents hydrogen, straight-chain or branched alkyl having 1 to 4 carbon atoms, or phenyl or benzyl which is in each case optionally mono- to trisubstituted by identical or different substituents, suitable substituents preferably being the phenyl substituents already mentioned in the case of R;

$R^2$ represents hydrogen or straight-chain or branched alkyl having 1 to 4 carbon atoms;

$R^3$ represents hydrogen, straight-chain or branched alkyl having 1 to 6 carbon atoms, straight-chain or branched hydroxyalkyl having 1 to 4 carbon atoms, alkoxycarbonylalkyl having 1 to 4 carbon atoms in both the alkoxy part and in the alkyl part, hydroxycarbonylalkyl and aminocarbonylalkyl in each case having 1 to 4 carbon atoms in the alkyl part, 1,2,4-triazol-1-ylalkyl, 1,2,4-triazol-4-ylalkyl, imidazol-4-ylalkyl and pyrazol-1-ylalkyl in each case having 1 to 6 carbon atoms in the alkyl part, cyanoalkyl having 1 to 4 carbon atoms, alkenyl or alkinyl in each case having 2 to 4 carbon atoms, cycloalkyl, having 3 to 7 carbon atoms, which is optionally mono- to trisubstituted by identical or different substituents, substituents which may preferably be mentioned being halogen and alkyl having 1 to 4 carbon atoms, furthermore represents phenyl or phenylalkyl, having 1 to 4 carbon atoms in the alkyl part, which is optionally mono- to trisubstituted in the phenyl part by identical or different substituents, suitable substituents in each case preferably being the phenyl substituents already mentioned in the case of R, or represents the $R^4–SO_n–Z$ group,

where

$R^4$ represents hydrogen, straight-chain or branched alkyl having 1 to 4 carbon atoms, phenylalkyl, having 1 to 4 carbon atoms in the alkyl part, which is optionally mono- to trisubstituted in the phenyl part by identical or different substituents, suitable substituents being the phenyl substituents already mentioned in the case of R;

n represents the numbers 0, 1 or 2, and

Z represents a straight-chain or branched alkylene chain having 1 to 4 carbon atoms;

$R^1$ and $R^3$, together with the nitrogen atom and the carbon atom to which they are bound, represent a 5- or 6-membered heterocycle;

$R^2$ and $R^3$, together with the carbon atom to which they are bound, represent cycloalkylidene having 3 to 6 carbon atoms;

X represents the $-OR^I$ or $-NR^{II}R^{III}$ groups,
where
$R^I$ represents hydrogen, straight-chain or branched alkyl having 1 to 4 carbon atoms, or alkenyl or alkinyl in each case having 2 to 4 carbon atoms;
$R^{II}$ represents hydrogen or straight-chain or branched alkyl having 1 to 4 carbon atoms;
$R^{III}$ represents hydrogen, straight-chain or branched alkyl having 1 to 4 carbon atoms, alkenyl or alkinyl in each case having 2 to 4 carbon atoms, halogenoalkyl having 1 to 4 carbon atoms and 1 to 5 identical or different halogen atoms, alkoxyalkyl having 1 to 4 carbon atoms in both the alkoxy part and in the alkyl part, dialkylaminoalkyl having up to 4 carbon atoms in the individual alkyl parts, alkoxycarbonylalkyl having 1 to 4 carbon atoms in both the alkoxy part and in the alkyl part, hydroxycarbonylalkyl having 1 to 4 carbon atoms in the alkyl part, aminocarbonylalkyl, alkylaminocarbonylalkyl or dialkylaminocarbonylalkyl in each case having 1 to 4 carbon atoms in each alkyl part cyanoalkyl having 1 to 4 carbon atoms in the alkyl part, phenylalkyl, having 1 to 4 carbon atoms in the alkyl part, which is optionally mono- to trisubstituted in the phenyl part by identical or different substituents, or phenyl which is mono- to trisubstituted by identical or different substituents, suitable substituents in each case being the phenyl substituents already mentioned in the case of R, and also represents cycloalkyl, having 3 to 7 carbon atoms, which is optionally substituted by identical or different substituents, suitable substituents preferably being halogen and alkyl having 1 to 4 carbon atoms; or
$R^{II}$ and $R^{III}$, together with the nitrogen atom to which they are bound, represent a 5- or 6-membered heterocycle, which may optionally contain oxygen or nitrogen as further heteroatoms and may optionally be substituted by cyano, halogen, alkyl having 1 to 4 carbon atoms, hydroxycarbonyl, alkoxycarbonyl having 1 to 4 carbon atoms in the alkoxy part, aminocarbonyl, and alkylaminocarbonyl and dialkylaminocarbonyl in each case having 1 to 4 carbon atoms in the individual alkyl parts, with the exception of the compounds N-(methoxycarbonylmethyl)-(2-cyano-2-methoximino)-acetamide and N-(carbamoylmethyl)-(2-cyano-2-methoximino)-acetamide.

2. Compounds of the general formula (I) according to Claim 1, where
R represents straight-chain or branched alkyl having 1 to 4 carbon atoms, allyl, propargyl, cyanomethyl, cyanoethyl, 1,2,4-triazol-1-ylalkyl and pyrazol-1-ylalkyl having 1 to 4 carbon atoms in the alkyl part, phenylalkyl, having 1 or 2 carbon atoms in the alkyl part, which is optionally mono- or disubstituted in the phenyl part by identical or different substituents, substituents which may be mentioned, in particular, being: fluorine, chlorine, cyano, nitro, hydroxyl, methyl, methoxy, methylthio, methylsulphinyl, methylsulphonyl and phenyl which is optionally mono- or disubstituted by identical or different fluorine or chlorine substituents; furthermore represents cyclopropyl, cyclobutyl, cyclopentyl, cyclohexyl or cycloheptyl which is in each case optionally mono- or disubstituted by identical or different fluorine, chlorine or methyl substituents;
$R^1$ represents hydrogen, methyl, ethyl, and phenyl or benzyl which is in each case optionally mono- or disubstituted by identical or different substituents, suitable substituents being, in particular, the phenyl substituents already mentioned in the case of R;
$R^2$ represents hydrogen, methyl or ethyl;
$R^3$ represents hydrogen, straight-chain or branched alkyl having 1 to 4 carbon atoms, hydroxymethyl, 1-hydroxyethyl, methoxycarbonylmethyl, methoxycarbonylethyl, ethoxycarbonylmethyl, ethoxycarbonylethyl, hydroxycarbonylmethyl, hydroxycarbonylethyl, aminocarbonylmethyl, aminocarbonylethyl, represents 1,2,4-triazol-1-ylalkyl, 1,2,4-triazol-4-ylalkyl, imidazol-4-ylalkyl and pyrazol-1-ylalkyl in each case having 1 to 4 carbon atoms in the alkyl part, cyanomethyl, cyanoethyl, allyl, propargyl, represents cyclopropyl, cyclobutyl, cyclopentyl, cyclohexyl or cycloheptyl which is in each case optionally mono- or disubstituted by identical or different fluorine, chlorine or methyl substituents; furthermore represents phenylalkyl, having 1 or 2 carbon atoms in the alkyl part, which is optionally mono- or disubstituted in the phenyl part by identical or different substituents, or represents phenyl which is mono- or disubstituted by identical or different substituents, suitable substituents being the phenyl substituents already mentioned in the case of R, and furthermore represents the $R^4-SO_n-Z$ group,
where
$R^4$ represents hydrogen, methyl, ethyl, or phenylalkyl, having 1 or 2 carbon atoms in the alkyl part, which is optionally mono- or disubstituted in the phenyl part by identical or different substituents, suitable substituents being, in particular, the phenyl substituents already mentioned in the case of R,
n represents the numbers 0, 1 or 2, and
Z represents a straight-chain or branched alkylene chain having 1 or 2 carbon atoms;
$R^1$ and $R^3$, together with the nitrogen atom and the carbon atom to which they are bound, represent a 5- to 6-membered heterocycle;
$R^2$ and $R^3$, together with the carbon atom to which they are bound, represent cyclopropylidene, and
X represents the $-OR^I$ or $-NR^{II}R^{III}$ groups,
where
$R^I$ represents hydrogen, methyl, ethyl, allyl and propargyl;
$R^{II}$ represents hydrogen, methyl or ethyl;
$R^{III}$ represents hydrogen, methyl, ethyl, n-propyl, i-propyl, n-butyl, i-butyl, sec.-butyl, tert.-butyl, allyl,

propargyl, halogenoalkyl having 1 or 2 carbon atoms and 1 to 3 identical or different halogen atoms, such as fluorine and chlorine atoms, alkoxyalkyl having 1 or 2 carbon atoms in both the alkoxy part and in the alkyl part, dialkylamino having 1 or 2 carbon atoms in the individual alkyl parts, alkoxycarbonylalkyl, hydroxycarbonylalkyl, aminocarbonylalkyl, alkylaminocarbonylalkyl or dialkylaminocarbonylalkyl in each case having 1 or 2 carbon atoms in each alkyl part, cyanoalkyl; furthermore represents phenylalkyl, having 1 or 2 carbon atoms in the alkyl part, which is optionally mono- or disubstituted in the phenyl part by identical or different substituents, phenyl which is mono -or disubstituted by identical or different substituents, suitable substituents in each case being the phenyl substituents already mentioned in the case of R, and also represents cyclopropyl, cyclobutyl, cyclopentyl, cyclohexyl or cycloheptyl which is in each case optionally mono- or disubstituted by identical or different fluorine, chlorine or methyl substituents;

$R^{II}$ and $R^{III}$, together with the nitrogen atom to which they are bound, represent a 5- or 6-membered heterocycle which may optionally contain oxygen or nitrogen as further hetero atoms and which may optionally be substituted by cyano, fluorine, chlorine, bromine, iodine, methyl, ethyl, i-propyl, hydroxycarbonyl, methoxycarbonyl, ethoxycarbonyl, aminocarbonyl, methylaminocarbonyl, ethylaminocarbonyl, dimethylaminocarbonyl, diethylaminocarbonyl and methylethylaminocarbonyl.

3. Process for the preparation of mixtures of E/Z isomers and pure Z isomers of $N^{\alpha}$-(2-cyano-2-alkoximinoacetyl)-amino acid derivatives and peptides of the formula (I)

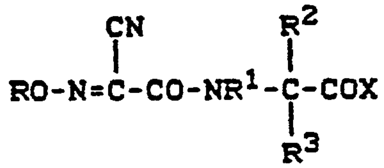

(I)

in which

R, $R^1$, $R^2$, $R^3$ and X have the meaning given in Claim 1, with the exception of the compounds N-(methoxycarbonylmethyl)-(2-cyano-2-methoximino)-acetamide and N-(carbamoylmethyl)-(2-cyano-2-methoximino)-acetamide, characterized in that the mixtures of E/Z isomers or the pure Z isomers of the 2-cyano-2-oximino-acetic acid derivatives of the general formula (II)

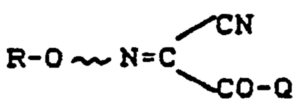

(II)

in which

R has the abovementioned meaning, and

Q represents chlorine or alkoxy having 1–4 C atoms, are reacted with amino acid derivatives of the general formula (III)

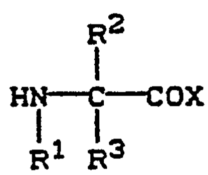

(III)

in which

$R^1$, $R^2$, $R^3$ and X have the abovementioned meanings, if appropriate in the presence of a catalyst, if appropriate in the presence of an acid-binding agent and if appropriate in the presence of a diluent.

4. Fungicidal agents, characterized in that they contain at least one compound of the general formula (I) according to Claim 1, with the exception of the compounds N-(methoxycarbonylmethyl)-(2-cyano-2-methoximino)-acetamide and N-(carbamoylmethyl)-(2-cyano-2-methoximino)-acetamide.

5. Process for combating fungal pests, characterized in that at least one compound of the formula (I) according to Claim 1 is allowed to act on fungal pests and/or on their habitat, with the exception of the compounds N-(methoxycarbonylmethyl)-(2-cyano-2-methoximino)-acetamide and N-(carbamoylmethyl)-(2-cyano-2-methoximino)-acetamide.

6. Use of compounds of the general formula (I) according to Claim 1 for combating fungal pests, with the exception of the compounds N-(methoxycarbonylmethyl)-(2-cyano-2-methoximino)-acetamide and N-(carbamoylmethyl)-(2-cyano-2-methoximino)-acetamide.

7. Process for the preparation of fungicidal agents, characterized in that compounds of the general formula (I) according to Claim 1 are mixed with extenders and/or surface-active agents, with the exception of the compounds N-(methoxycarbonylmethyl)-(2-cyano-2-methoximino)-acetamide and N-(carbamoylmethyl)-(2-cyano-2-methoximino)-acetamide.

**Revendications**

1. Mélanges d'isomères E/Z et isomères Z purs de peptides et de dérivés de $N^{\alpha}$-(2-cyano-2-alcoximino-acétyl)-amino-acides de formule générale (I)

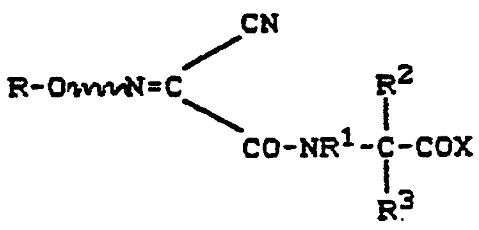

(I)

dans laquelle

R représente un groupe alkyle à chaîne droite ou ramifiée contenant 1 à 8 atomes de carbone, un groupe alcényle ou un groupe alcynyle contenant chacun 2 à 4 atomes de carbone, un groupe cyanalkyle contenant 1 à 4 atomes de carbone, un groupe 1,2,4-triazol-1-yl-alkyle et un groupe pyrazol-1-yl-alkyle contenant chacun 1 à 6 atomes de carbone dans la fraction alkyle, un groupe phénylalkyle éventuellement substitué une à trois fois de manière identique ou différente dans la fraction phényle et contenant 1 à 4 atomes de carbone dans la fraction alkyle en mentionnant, comme substituants, de préférence: un atome d'halogène, un groupe cyano, un groupe nitro, un groupe hydroxyle, un groupe alkyle, un groupe alcoxy, un groupe alkylsulfinyle, un groupe alkylsulfonyle et un groupe alkylthio contenant chacun 1 à 4 atomes de carbone et un groupe phényle éventuellement substitué une à trois fois de manière identique ou différente par un atome d'halogène, ou un groupe cycloalkyle éventuellement substitué une à trois fois de manière identique ou différente et contenant 3 à 7 atomes de carbone en mentionnant, comme substituants, de préférence, un atome d'halogène et un groupe alkyle contenant 1 à 4 atomes de carbone;

$R^1$ représente un atome d'hydrogène, un groupe alkyle à chaîne droite ou ramifiée contenant 1 à 4 atomes de carbone ou un groupe phényle ou benzyle chacun éventuellement substitué une à trois fois de manière identique ou différente en envisageant, comme substituants, de préférence, les substituants du phényle déjà mentionnés pour R;

$R^2$ représente un atome d'hydrogène ou un groupe alkyle à chaîne droite ou ramifiée contenant 1 à 4 atomes de carbone;

$R^3$ représente un atome d'hydrogène, un groupe alkyle à chaîne droite ou ramifiée contenant 1 à 6 atomes de carbone, un groupe hydroxyalkyle à chaîne droite ou ramifiée contenant 1 à 4 atomes de carbone, un groupe alcoxycarbonylalkyle contenant chaque fois 1 à 4 atomes de carbone dans la fraction alcoxy et dans la fraction alkyle, un groupe hydroxycarbonylalkyle et un groupe aminocarbonylalkyle contenant chacun 1 à 4 atomes de carbone dans la fraction alkyle, un groupe 1,2,4-triazol-1-yl-alkyle, un groupe 1,2,4-triazol-4-yl-alkyle, un groupe imidazol-4-yl-alkyle et un groupe pyrazol-1-yl-alkyle contenant chacun 1 à 6 atomes de carbone dans la fraction alkyle, un groupe cyanalkyle contenant 1 à 4 atomes de carbone, un groupe alcényle ou un groupe alcynyle contenant chacun 2 à 4 atomes de carbone, un groupe cycloalkyle éventuellement substitué une à trois fois de manière identique ou différente et contenant 3 à 7 atomes de carbone en mentionnant, comme substituants, de préférence, un atome d'halogène et un groupe alkyle contenant 1 à 4 atomes de carbone, de même qu'un groupe phényle ou phénylalkyle éventuellement substitué une à trois fois de manière identique ou différente dans la fraction phényle et contenant 1 à 4 atomes de carbone dans la fraction alkyle en envisageant, chaque fois, comme substituants, de préférence, les substituants du phényle déjà mentionnés pour R, ou le groupement $R^4$–$SO_n$–Z–,

$R^4$ représentant un atome d'hydrogène, un groupe alkyle à chaîne droite ou ramifiée contenant 1 à 4 atomes de carbone, un groupe phénylalkyle éventuellement substitué dans la fraction phényle une à trois fois de manière identique ou différente et contenant 1 à 4 atomes de carbone dans la fraction alkyle en envisageant, comme substituants, les substituants du phényle déjà mentionnés pour R;

n représente les nombres 0, 1 ou 2, et Z représente une chaîne alkylène droite ou ramifiée contenant 1 à 4 atomes de carbone;

$R^1$ et $R^3$, ensemble avec l'atome d'azote et l'atome de carbone auxquels ils sont reliés, représentent un hétérocycle pentagonal ou hexagonal;

$R^2$ et $R^3$, ensemble avec l'atome de carbone auquel ils sont reliés, représentent un groupe cycloalkylidè-

ne contenant 3 à 6 atomes de carbone;
X représente les groupements $-OR^I$ ou $-NR^{II}R^{III}$,
$R^I$ représentant un atome d'hydrogène, un groupe alkyle à chaîne droite ou ramifiée contenant 1 à 4 atomes de carbone, un groupe alcényle ou un groupe alcynyle contenant chacun 2 à 4 atomes de carbone;
$R^{II}$ représentant un atome d'hydrogène ou un groupe alkyle à chaîne droite ou ramifiée contenant 1 à 4 atomes de carbone;
$R^{III}$ représentant un atome d'hydrogène, un groupe alkyle à chaîne droite ou ramifiée contenant 1 à 4 atomes de carbone, un groupe alcényle ou un groupe alcynyle contenant chacun 2 à 4 atomes de carbone, un groupe halogénalkyle contenant 1 à 4 atomes de carbone et 1 à 5 atomes d'halogènes identiques ou différents, un groupe alcoxyalkyle contenant chaque fois 1 à 4 atomes de carbone dans la fraction alcoxy et dans la fraction alkyle, un groupe dialkylaminoalkyle contenant jusqu'à 4 atomes de carbone dans les fractions alkyle individuelles, un groupe alcoxycarbonylalkyle contenant chaque fois 1 à 4 atomes de carbone dans la fraction alcoxy et dans la fraction alkyle, un groupe hydroxycarbonylalkyle contenant 1 à 4 atomes de carbone dans la fraction alkyle, un groupe aminocarbonylalkyle, un groupe alkylaminocarbonylalkyle ou un groupe dialkylaminocarbonylalkyle contenant chaque fois 1 à 4 atomes de carbone dans chaque fraction alkyle, un groupe cyanalkyle contenant 1 à 4 atomes de carbone dans la fraction alkyle, un groupe phénylalkyle éventuellement substitué une à trois fois de manière identique ou différente dans la fraction phényle et contenant 1 à 4 atomes de carbone dans la fraction alkyle, ou un groupe phényle substitué une à trois fois de manière identique ou différente, en envisageant, comme substituants, chaque fois les substituants du phényle déjà mentionnés pour R, de même qu'un groupe cycloalkyle éventuellement substitué de manière identique ou différente et contenant 3 à 7 atomes de carbone en envisageant, comme substituants, de préférence, un atome d'halogène et un groupe alkyle contenant 1 à 4 atomes de carbone;
ou
$R^{II}$ et $R^{III}$, ensemble avec l'atome d'azote auquel ils sont reliés, représentent un hétérocycle pentagonal ou hexagonal pouvant éventuellement contenir un atome d'oxygène ou un atome d'azote comme autres hétéro-atomes et pouvant éventuellement être substitué par un groupe cyano, un atome d'halogène, un groupe alkyle contenant 1 à 4 atomes de carbone, un groupe hydroxycarbonyle, un groupe alcoxycarbonyle contenant 1 à 4 atomes de carbone dans la fraction alcoxy, un groupe aminocarbonyle, ainsi qu'un groupe alkylaminocarbonyle et un groupe dialkylaminocarbonyle contenant chacun 1 à 4 atomes de carbone dans les fractions alkyle individuelles, à l'exception des composés N-(méthoxycarbonylméthyl)-(2-cyano-2-méthoximino)-acétamide et N-(carbamoylméthyl)-(2-cyano-2-méthoximino)-acétamide.

2. Composés de formule générale (I) selon la revendication 1, où
R représente un groupe alkyle à chaîne droite ou ramifiée contenant 1 à 4 atomes de carbone, un groupe allyle, un groupe propargyle, un groupe cyanométhyle, un groupe cyanéthyle, un groupe 1,2,4-triazol-1-yl-alkyle et un groupe pyrazol-1-yl-alkyle contenant 1 à 4 atomes de carbone dans la fraction alkyle, un groupe phénylalkyle éventuellement substitué dans la fraction phényle une ou deux fois de manière identique ou différente et contenant 1 ou 2 atomes de carbone dans la fraction alkyle, en mentionnant, comme substituants, en particulier: un atome de fluor, un atome de chlore, un groupe cyano, un groupe nitro, un groupe hydroxyle, un groupe méthyle, un groupe méthoxy, un groupe méthylthio, un groupe méthylsulfinyle, un groupe méthylsulfonyle et un groupe phényle éventuellement substitué une ou deux fois de manière identique ou différente par un atome de fluor ou un atome de chlore; de même qu'un groupe cyclopropyle, un groupe cyclobutyle, un groupe cyclopentyle, un groupe cyclohexyle ou un groupe cycloheptyle éventuellement substitués chacun une ou deux fois de manière identique ou différente par un atome de fluor, un atome de chlore ou un groupe méthyle;
$R^1$ représente un atome d'hydrogène, un groupe méthyle, un groupe éthyle, de même qu'un groupe benzyle ou un groupe phényle chacun éventuellement substitués une ou deux fois de manière identique ou différente en envisageant, comme substituants, en particulier, les substituants du phényle déjà mentionnés pour R;
$R^2$ représente un atome d'hydrogène, un groupe méthyle ou un groupe éthyle;
$R^3$ représente un atome d'hydrogène, un groupe alkyle à chaîne droite ou ramifiée contenant 1 à 4 atomes de carbone, un groupe hydroxyméthyle, un groupe 1-hydroxyéthyle, un groupe méthoxycarbonylméthyle, un groupe méthoxycarbonyléthyle, un groupe éthoxycarbonylméthyle, un groupe éthoxycarbonyléthyle, un groupe hydroxycarbonylméthyle, un groupe hydroxycarbonyléthyle, un groupe aminocarbonylméthyle, un groupe aminocarbonyléthyle, un groupe 1,2,4-triazol-1-yl-alkyle, un groupe 1,2,4-triazol-4-yl-alkyle, un groupe imidazol-4-yl-alkyle et un groupe pyrazol-1-yl-alkyle contenant chacun 1 à 4 atomes de carbone dans la fraction alkyle, un groupe cyanométhyle, un groupe cyanéthyle, un groupe allyle, un groupe propargyle, un groupe cyclopropyle, un groupe cyclobutyle, un groupe cyclopentyle, un groupe cyclohexyle ou un groupe cycloheptyle éventuellement substitués chacun une ou deux fois de manière identique ou différente par un atome de fluor, un atome de chlore ou un groupe méthyle, de même qu'un groupe phénylalkyle éventuellement substitué une ou deux fois de manière identique ou différente dans la fraction phényle et contenant 1 ou 2 atomes de carbone dans la fraction alkyle, ou un groupe phényle substitué une ou deux fois de manière identique ou différente en envisageant, comme substituants, les substituants du phényle déjà mentionnés pour R, de même que le groupement $R^4-SO_n-Z$,

$R^4$ représente un atome d'hydrogène, un groupe méthyle, un groupe éthyle ou un groupe phénylalkyle éventuellement substitué une ou deux fois de manière identique ou différente dans la fraction phényle et contenant 1 ou 2 atomes de carbone dans la fraction alkyle en envisageant, comme substituants, en particulier, les substituants du phényle déjà mentionnés pour R,
n représente les nombres 0, 1 ou 2, et
Z représente une chaîne alkylène droite ou ramifiée contenant 1 ou 2 atomes de carbone;
$R^1$ et $R^3$, ensemble avec l'atome d'azote et l'atome de carbone auxquels ils sont reliés, représentent un hétérocycle pentagonal ou hexagonal;
$R^2$ et $R^3$, ensemble avec l'atome de carbone auquel ils sont reliés, représentent un groupe cyclopropylidène, et
X représente les groupements $-OR^I$ ou $-NR^{II}R^{III}$,
$R^I$ représentant un atome d'hydrogène, un groupe méthyle, un groupe éthyle, un groupe allyle, de même qu'un groupe propargyle;
$R^{II}$ représente un atome d'hydrogène, un groupe méthyle ou un groupe éthyle;
$R^{III}$ représente un atome d'hydrogène, un groupe méthyle, un groupe éthyle, un groupe n-propyle, un groupe i-propyle, un groupe n-butyle, un groupe i-butyle, un groupe sec.-butyle, un groupe tert.-butyle, un groupe allyle, un groupe propargyle, un groupe halogénalkyle contenant 1 ou 2 atomes de carbone et 1 à 3 atomes d'halogènes identiques ou différents tels que des atomes de fluor et de chlore, un groupe alcoxyalkyle contenant chaque fois 1 ou 2 atomes de carbone dans la fraction alcoxy et dans la fraction alkyle, un groupe dialkylamino-alkyle contenant 1 ou 2 atomes de carbone dans les fractions alkyle individuelles, un groupe alcoxy-carbonylalkyle, un groupe hydroxycarbonylalkyle, un groupe aminocarbonylalkyle, un groupe alkylaminocarbonylalkyle ou un groupe dialkylaminocarbonylalkyle contenant chacun 1 ou 2 atomes de carbone dans chaque fraction alkyle, un groupe cyano-alkyle; de même qu'un groupe phénylalkyle éventuellement substitué ou deux fois de manière identique ou différente dans la fraction phényle et contenant 1 ou 2 atomes de carbone dans la fraction alkyle, un groupe phényle substitué une ou deux fois de manière identique ou différente, en envisageant, comme substituants, chaque fois les substituants du phényle déjà mentionnés pour R, de même qu'un groupe cyclopropyle, un groupe cyclobutyle, un groupe cyclopentyle, un groupe cyclohexyle ou un groupe cycloheptyle chaque fois éventuellement substitué une ou deux fois de manière identique ou différente par un atome de fluor, un atome de chlore ou un groupe méthyle;
$R^{II}$ et $R^{III}$ représentent, ensemble avec l'atome d'azote auquel ils sont reliés, un hétérocycle pentagonal ou hexagonal pouvant éventuellement contenir un atome d'oxygène ou un atome d'azote comme autres hétéro-atomes et qui peut être éventuellement substitué par un groupe cyano, un atome de fluor, un atome de chlore, un atome de brome, un atome d'iode, un groupe méthyle, un groupe éthyle, un groupe i-propyle, un groupe hydroxycarbonyle, un groupe méthoxycarbonyle, un groupe éthoxycarbonyle, un groupe aminocarbonyle, un groupe méthylaminocarbonyle, un groupe éthylaminocarbonyle, un groupe diméthylaminocarbonyle, un groupe diéthylaminocarbonyle, ainsi qu'un groupe méthyléthylaminocarbonyle.

3. Procédé de préparation de mélanges d'isomères E/Z et d'isomères Z purs de peptides et de dérivés de $N^{\alpha}$-(2-cyano-2-alcoximino-acétyl)-amino-acides de formule (I):

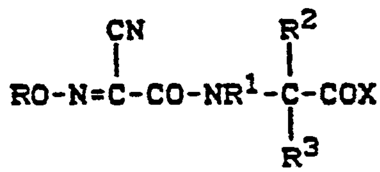

(I)

dans laquelle
R, $R^1$, $R^2$, $R^3$ et X ont les significations indiquées dans la revendication 1, à l'exception des composés N-(méthoxycarbonylméthyl)-(2-cyano-2-méthoximino)-acétamide et N-(carbamoylméthyl)-(2-cyano-2-méthoximino)-acétamide, caractérisé en ce qu'on fait réagir les mélanges d'isomères E/Z ou les isomères Z purs des dérivés de l'acide 2-cyano-2-oximino-acétique de formule générale (II):

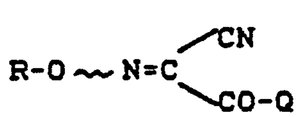

(II)

dans laquelle
R a la signification indiquée ci-dessus, et
Q représente un atome de chlore ou un groupe alcoxy contenant 1 à 4 atomes de carbone, avec des dérivés d'amino-acides de formule générale (III):

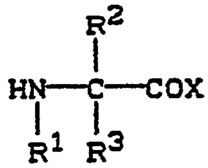

(III)

dans laquelle

$R^1$, $R^2$, $R^3$ et X ont les significations indiquées ci-dessus, éventuellement en présence d'un catalyseur, éventuellement en présence d'un agent fixateur d'acide et éventuellement en présence d'un diluant.

4. Agents fongicides, caractérisés en ce qu'ils contiennent au moins un composé de formule générale (I) selon la revendication 1, à l'exception des composés N-(méthoxycarbonylméthyl)-(2-cyano-2-méthoximino)-acétamide et N-(carbamoylméthyl)-(2-cyano-2-méthoximino)-acétamide.

5. Procédé en vue de combattre les parasites fongiques, caractérisé en ce qu'on fait agir au moins un composé de formule (I) selon la revendication 1 sur des parasites fongiques et/ou leur biotope, à l'exception des composés N-(méthoxycarbonylméthyl)-(2-cyano-2-méthoximino)-acétamide et N-(carbamoylméthyl)-(2-cyano-2-méthoximino)-acétamide.

6. Utilisation de composés de formule générale (I) selon la revendication 1 en vue de combattre les parasites fongiques, à l'exception des composés N-(méthoxycarbonyl-méthyl)-(2-cyano-2-méthoximino)-acétamide.

7. Procédé de préparation d'agents fongicides, caractérisé en ce qu'on mélange des composés de formule générale (I) selon la revendication 1 avec des diluants et/ou des agents tensioactifs, à l'exception des composés N-(méthoxycarbonylméthyl)-(2-cyano-2-méthoximino)-acétamide et N-(carbamoylméthyl)-(2-cyano-2-méthoximino)-acétamide.